Europäisches Patentamt

European Patent Office

Office européen des brevets

(11) Veröffentlichungsnummer: **0 262 501**
**A1**

# (12) EUROPÄISCHE PATENTANMELDUNG

(21) Anmeldenummer: 87113409.4

(51) Int. Cl.⁴: **A61B 6/00**

(22) Anmeldetag: 14.09.87

(30) Priorität: 26.09.86 DE 3632788

(43) Veröffentlichungstag der Anmeldung:
06.04.88 Patentblatt 88/14

(84) Benannte Vertragsstaaten:
**DE FR GB IT**

(71) Anmelder: **Siemens Aktiengesellschaft Berlin
und München**
**Wittelsbacherplatz 2**
**D-8000 München 2(DE)**

(72) Erfinder: **Heubeck, Erich**
**Blütenweg 11**
**D-6140 Bensheim(DE)**
Erfinder: **Molitor, Dieter**
**Kurt-Schumacher-Strasse 9**
**D-6842 Bürstadt(DE)**
Erfinder: **Schmitt, Heinrich**
**Drususstrasse 9**
**D-6140 Bensheim(DE)**

(54) **Vorrichtung zur Positionierung eines Patientenkopfes zur Erstellung von Fernröntgenaufnahmen.**

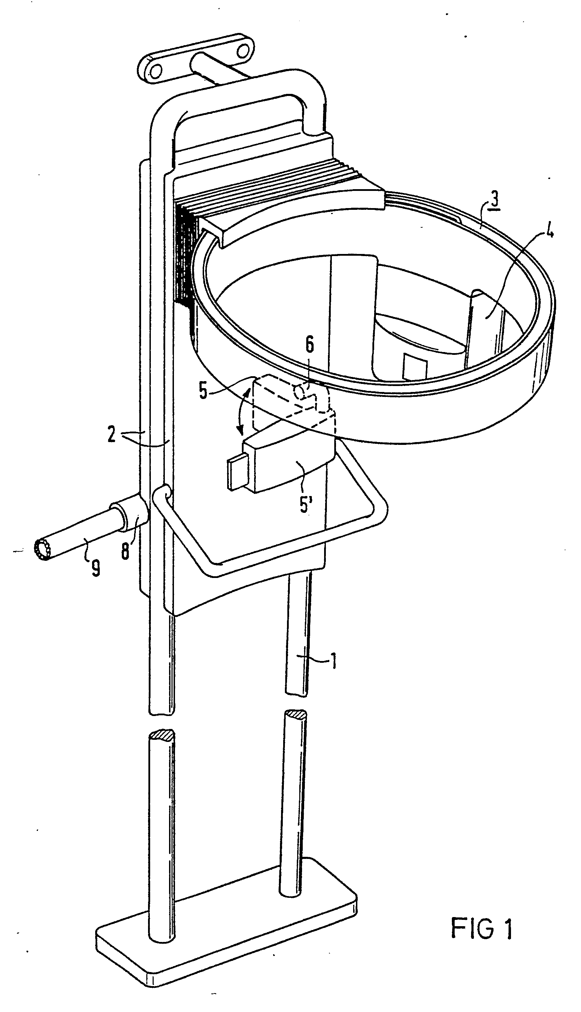

(57) Die Erfindung betrifft eine Vorrichtung zur Positionierung eines Patientenkopfes zur Erstellung von Fernröntgenaufnahmen vom Schädel eines Patienten. Sie enthält einen mittels eines Querträgers (9) an ein Röntgendiagnostikgerät (Fig. 1) zur Erstellung von Schichtaufnahmen vom Kiefer eines Patienten adaptierbaren Schädelhalter (10), der in bekannter Weise verstellbare Ohrolivenhalter (12), ein zumindest in Höhe und Tiefe verstellbares Anlageteil (13) für das Nasion des Patienten sowie ein in Richtung auf die Strahlenquelle (4) des Röntgendiagnostikgerätes (Fig. 1) verstellbares Aufnahmeteil (17) für eine Filmkassette (18) enthält. Um insbesondere reproduzierbare Kiefergelenksaufnahmen machen zu können, ist erfindungsgemäß der Schädelhalter (10) mittels eines Tragteils (14) am Querträger (9) um eine horizontale Achslagerung (25) derart schwenkbar gehalten ist, daß der Patientenkopf in bezug auf die Saggittalebene in verschiedenen Neigungsstellungen (14') einstellbar ist.

FIG 2

## Vorrichtung zur Positionierung eines Patientenkopfes zur Erstellung von Fernröntgenaufnahmen

Die Erfindung bezieht sich auf eine Vorrichtung zur Positionierung eines Patientenkopfes zur Erstellung von Fernröntgenaufnahmen vom Schädel eines Patienten (sog. Ceph-Aufnahmen) enthaltend einen Querträger, dessen eines Ende an einem Röntgendiagnostikgerät zur Erstellung von Panoramaschichtaufnahmen vom Kiefer eines Patienten adaptierbar ist und an dessen anderem Ende ein Schädelhalter angeordnet ist, der ein um eine lotrechte Achse drehbares Teil enthält, an dem einerseits zwei verstellbare Ohrolivenhalter und andererseits ein zumindest in Höhe und Tiefe verstellbares Anlageteil am Nasion des Patienten angeordnet sind, und der ferner ein in Richtung auf die Strahlenquelle des Röntgendiagnostikgerätes verstellbares Aufnahmeteil für eine Filmkassette enthält.

Eine Vorrichtung dieser Art ist beispielsweise mit der Bezeichnung ORTHOCEPH 10 bekannt. Mit einer solchen Vorrichtung lassen sich sehr gut verschiedene Schädelaufnahmen z.B. von vorne nach hinten und umgekehrt (sog. p.a-oder a.p-Aufnahmen) sowie seitliche Aufnahmen erstellen.

Der im Patentanspruch 1 angegebenen Erfindung liegt die Aufgabe zugrunde, eine Vorrichtung der eingangs genannten Gattung dahingehend zu verbesssern, daß man außer zu den bereits erwähnten Schädelaufnahmen auch Spezialaufnahmen von den Kiefergelenken erstellen kann und zwar in reproduzierbarer Weise. Solche Spezialkiefergelenksaufnahmen erfordern die genaue Lage des Kondylus in bezug auf die Röntgenstrahlen zu erfassen und dementsprechend den Patientenkopf einzustellen. Zur Bestimmung der genauen Lage der Kondylen sind in der Regel zwei Aufnahmen erforderlich, eine sogenannte Submento-Vertikel-Aufnahme, mit der der Kondylenachsenwinkel bestimmt werden kann, und eine zweite, sogenannte a.p.-Aufnahme, mit der der Schnittwinkel der Kondylenachsen in saggitaler Richtung bestimmt werden kann.

Ein wesentlicher Vorteil der erfindungsgemäßen Vorrichtung ist, daß sie an einem Panoramaröntgengerät adaptierbar ist und somit insbesondere die Kiefergelenke betreffende Schicht-und statische Aufnahmen an "einem Gerät" erstellt werden können. Die vorhandenen Freiheitsgrade erlauben die Positionierung des Patientenkopfes für Aufnahmen in zwei senkrecht zueianander stehenden Ebenen, und zwar sowohl in transmaxillärer Richtung (d.h. senkrecht zur Kondylenachse) als auch in transkranialer Richtung (d.h. parallel zur Kondylenachse bzw. entlang des Kiefergelenkspaltes).

Dazu ist es erforderlich, daß der Schädelhalter um eine horizontale Achslagerung geschwenkt werden kann, wobei durch die Ohrolivenhalter und das Nasionabstützelement der Patientenkopf für reproduzierbare Aufnahmen exakt fixiert ist. Der Patientenkopf kann durch Neigen und Drehen so positioniert werden, daß mit ein und demselben Gerät sowohl die eingangs erwähnten Standardaufnahmen als auch die weiters genannten Spezialkiefergelenksaufnahmen auf einfache Weise erstellt werden können.

Solche Spezialkiefergelenksaufnahmen lassen sich mit einem anderen bekannten Gerät (DE-PS 23 35918), welches nicht dazu geeignet ist, an ein Röntgendiagnostikgerät zur Erstellung von Panoramaschichtaufnahmen adaptiert zu werden, welches vielmehr ein eigenständiges Röntgengerät darstellt, insofern nicht erzielen, als der Patientenkopf, der ausschließlich über Ohrbolzenhalterungen fixiert ist, um die Achse der Ohrbolzenhalterungen neigbar ist, wodurch trotz der vielen Verstellmöglichkeiten, die dort angegeben sind, sich insbesondere keine reproduzierbaren Kiefergelenksaufnahmen erstellen lassen. Außerdem sind die erläuterten Verstelleinrichtungen, insbesondere die Doppelschlittenanordnung für den Kopfhalter, vergleichsweise aufwendig.

Demgegenüber stellt die erfindungsgemäße Vorrichtung eine konstruktiv wesentlich einfachere Lösung dar. Die den Schädelhalter aufnehmende Tragstange ist vorteilhafterweise in der Höhe einstellbar, wodurch sich die Möglichkeit eröffnet, ohne Formatänderung auch den letzten Halswirbel auf dem Film aufnehmen zu können. Zur Höhenverstellung ist vorteilhafterweise ein Teleskoprohr vorgesehen, welches zweckmäßigerweise durch einen Faltenbalg verkleidet ist. Das Tragrohr ist vorteilhafterweise an einem am Querträger befestigten Gehäuse verstellbar gehalten, welches gleichzeitig Führungsbuchsen zur Aufnahme von Führungselementen als Träger für eine Filmkassettenhalterung enthält.

Weitere vorteilhafte Ausgestaltungen und Weiterbildungen der Erfindung sind in den weiteren Unteransprüchen angegeben und aus der nachfolgenden Beschreibung eines Ausführungsbeispieles entnehmbar.

Es zeigen:

Figur 1 ein zahnärztliches Röntgendiagnostikgerät zur Erstellung von Panoramaschichtaufnahmen vom Kiefer eines Patienten in schaubildlicher Darstellung,

Figur 2 eine Ausführungsform der erfindungsgemäßen Vorrichtung die dazu geeignet ist an das in Fig. 1 gezeigte Gerät adaptiert zu werden.

Figuren 3, 4 und 5 Teile der in Fig. 2 gezeigten Vorrichtung in Grund-, Auf-und Seitenriß teilweise im Schnitt.

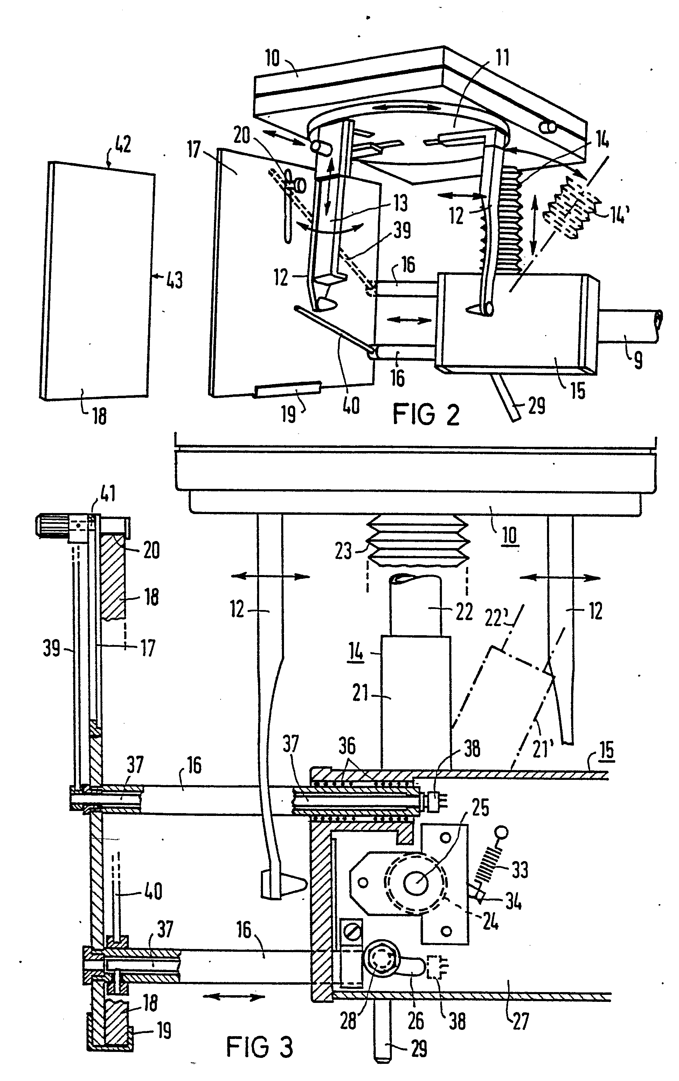

Die Fig. 1 zeigt in einer schaubildlichen Darstellung eine mögliche Ausführungsform eines zahnärztlichen Röntgendiagnostikgerätes wie sie zur Erstellung von Panoramaschichtaufnahmen vom Kiefer eines Patienten vorgesehen wird. Das Gerät enthält ein aus zwei Standrohren gebildetes Stativ (1), an dem ein Laufwagen (2) höhenverstellbar gehalten ist. Am Laufwagen (2) ist eine allgemein mit 3 bezeichnete Dreheinheit in Form eines geschlossenen Ringes gehalten, die einerseits einen Röntgenstrahler (4) und diametral dazu eine Filmkassettenhalterung (5) für Panoramaschichtaufnahmen enthält. Während der Röntgenstrahler (4) am Drehring (3) drehfest angeordnet ist, ist der Filmkassettenhalter (5) an einem abgewinkelten Tragarm (6) in Pfeilrichtung - schwenkbar gehalten. Der Filmkassettenhalter (5) kann so aus der gestrichelt eingezeichneten Gebrauchsstellung, die für normale Schichtaufnahmen geeignet ist, in die mit durchgehenden Linien gezeichnete Nicht-Gebrauchsstellung gebracht werden. In dieser Nicht-Gebrauchsstellung ist das Röntgengerät zusammen mit der nachfolgend anhand der Fig. 2 bis 5 erläuterten Vorrichtung für sog. Ceph-Aufnahmen, d.h. zur Erstellung von Fernaufnahmen vom Schädel eines Patienten geeignet. Um solche Fernaufnahmen machen zu können, muß in einer bestimmten Entfernung von etwa 1 bis 2 m vom Röntgenstrahler (4) eine in Fig. 2 gezeigte Kopf-und Kassenttenhalterung gehalten werden. Zu diesem Zweck ist an der Rückseite des Laufwagens (2) ein Adapter (8) vorgesehen, an dem die in den nachfolgenden Figuren noch näher erläuterte Kopfpositionier-und Kassettenhalterungsvorrichtung mittels eines Querträgers (9) adaptiert werden kann.

Die in Fig. 2 schaubildlich dargestellte Vorrichtung enthält einen Schädelhalter (10), an dem in bekannter Weise ein Drehteller (11) drehbar gehalten ist, an dem einerseits zwei diametral einander gegenüberliegende, in Pfeilrichtung verstellbare Ohrolivenhalter (12) und andererseits mit diesen in der Draufsicht etwa ein Dreieck bildendes Nasionabstützteil (13), welches in Höhe und Tiefe (in angegebener Pfeilrichtung) verstellbar sowie um eine Achslagerung schwenkbar am Drehteller (11) gehalten ist.

Der Schädelhalter (10) ist mittels eines vertikal verlaufenden Tragteils (14) mit dem Querträger verbunden. Mit (15) ist ein Gehäuse bezeichnet, an dem, wie anhand der nachfolgenden Figuren noch näher erläutert, einerseits Querträger (9) und Tragteil (14) und andererseits zwei horizontale Tragstangen (16) als Träger für eine Filmkassettenhalterung (17) gehalten sind. Die beiden Tragstangen (16) sind in der angegebenen Pfeilrichtung verstellbar im Gehäuse (15) gehalten. Die den Röntgenfilm enthaltende Filmkassette (18) wird senkrecht stehend in die Filmkassettenhalterung (17) eingelegt, wozu entsprechende Führungsglieder (19, 20) vorhanden sind.

Die Fig. 3 zeigt die Vorrichtung nach Fig. 2 in einer Frontansicht teilweise im Schnitt. Das Tragteil (14) besteht aus zwei teleskopartig ineinander greifenden Rohren (21, 22) die durch einen gemeinsamen Faltenbalg (23) abgedeckt sind. Um verschiedene Ausziehlängen und damit eine Höhenverstellung des Schädelhalters gegenüber dem Gehäuse (15) zu erzielen, ist eines der beiden Rohre mit einer Längsnut und die andere mit einer Bohrung versehen, durch die eine Feststellschraube (24) (siehe Seitenansicht gemäß Fig. 5) greift. Neben der Höhenverstellbarkeit, die insbesondere dazu dient, bei Spezialaufnahmen auch den letzten Halswirbel mit abzubilden, ist der gesamte Schädelhalter (10) noch um eine horizontale Achslagerung (25) schwenkbar, wie dies durch die gestrichelte Darstellung des Tragteils (14) in Fig. 2 sowie der Teleskoprohre (21, 22) in Fig. 3 angegeben ist. Der Neigungswinkel ist begrenzt durch eine Nut (26) in der Gehäusewandung (27) und einen durch die Nut hindurchgreifenden Gewindebolzen (28) welcher, vgl. Fig. 5, das untere Ende des Teleskoprohrs (21) durchdringt und dort entsprechend befestigt ist. Am Gewindebolzen (28) ist ferner ein Spannhebel (29) aufgeschraubt, bei dessen Betätigung das Tragrohr (21) und somit das gesamte Tragteil (14) mit dem Gehäuse (15) verspannt und damit arretiert wird.

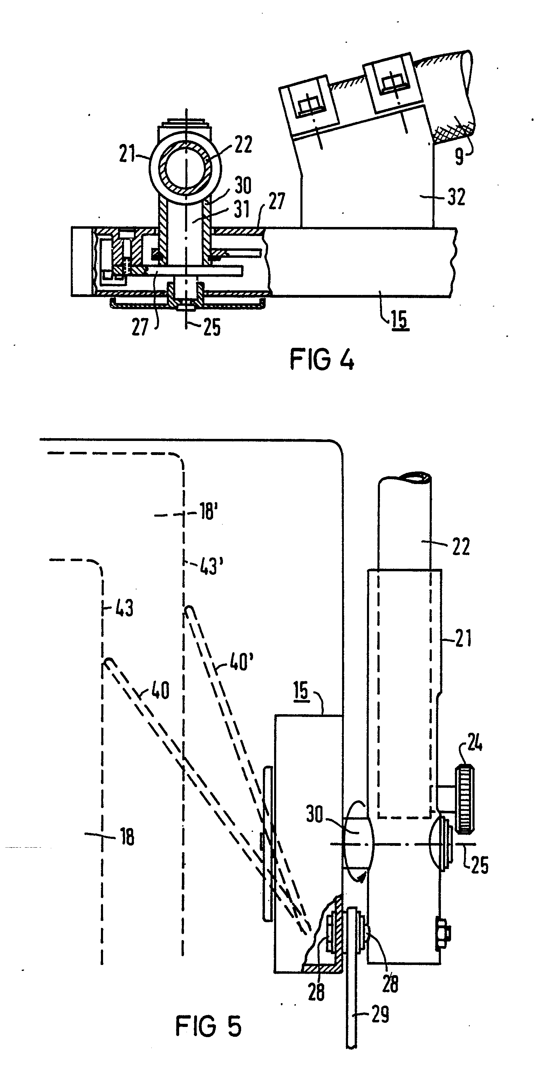

Wie insbesondere aus Fig. 4, die das Gehäuse (15) teilweise im Schnitt, in der Draufsicht zeigt, ersichtlich, erfolgt die Lagerung des Tragteils (14) über eine Buchse (30), die das untere Teleskoprohr (21) quer zu dessen Längsachse durchdringt und mit diesem fest verbunden ist und eines in der Buchse geführten Lagerzapfens (31), der an der Gehäusewandung (27) befestigt ist und um den sich die Buchse (30) drehen kann. Der Querträger (9) ist an einem Ansatz (32) des Gehäuses (15) derart befestigt, daß dessen Längsachse unter einem relativ kleinen spitzen Winkel zu der Ebene der Gehäusewandung verläuft.

Damit der Schädelhalter beim Öffnen des Spannhebels (29) seine Grundstellung beibehält, ist im Gehäuse (15) eine Zugfeder (33) vorgesehen, die einerseits mit dem Gehäuse (15) und andererseits mit einem mit der Buchse (30) win kelfest verbundenen, zur Drehachse (25) einen Hebelarm bildenden Abstützteil (34) verbunden ist.

Die beiden Tragstangen (16) sind als Rohre ausgebildet und mittels Lager (36) leichtgängig geführt. Im Inneren der rohrförmigen Stangen (16) sind Übertragungselemente (37) in Form von ebenfalls Stangen oder Hülsen gelagert, deren eine Enden mit einem Winkelgeber in Form eines Potentiometers (38) und deren andere Enden mit Tasthebeln (39, 40) winkelsteif verbunden sind, von denen der mit (39) bezeichnete Tasthebel hinter, und der mit (40) bezeichnete Tasthebel vor der Filmkassettenhalterung (17) angeordnet ist (siehe hierzu auch Fig. 2). Die beiden Tasthebel (39, 40) sind Teil einer Detektoranordnung, mit der selbsttätig das vorgesehene Filmkassettenformat beim Aufschieben auf die Filmkassettenhalterung (17) bestimmt und ggf. auf einem Display angezeigt wird, bzw. mit deren Signalen eine dem gewählten Filmkassettenformat zugehörige Primärblende automatisch in den Strahlengang zwischen Strahlenquelle und Objekt gebracht wird.

Aus Fig. 3 ist ersichtlich, daß der Tasthebel (39) einerseits mit der die Filmkassettenhalterung (17) durchdringenden Übertragungsstange (37) und andererseits mittels einer weiteren, die Filmkassettenhalterung (17) durchdringender Querverbindung (41) welches das als Tastkopf wirkende Führungsglied (20) enthält verbunden ist. Während letzteres (20) auf einer die Oberkante des Filmkassettenformates bestimmenden Referenzfläche (42) aufliegt, liegt das freie Ende des Tasthebels (40) direkt an einer die Breite des gewählten Filmkassettenformats entsprechenden Referenzfläche (43) an. (Fig. 2 und 5). Der jeweilige Schwenkwinkel der beiden Tasthebel (39, 40) entsprechend der Anlage ihrer Enden an den Referenzflächen (42, 43) der aufgesetzten Filmkassette bestimmt das gewählte Filmkassettenformat.

Bei entsprechender Verarbeitung der aus den Winkelgebern (38) gewonnenen Signale kann darüberhinaus anzeigt werden, daß eine Filmkassette eingelegt ist.

**Ansprüche**

1. Vorrichtung zur Positionierung eines Patientenkopfes zur Erstellung von Fernröntgenaufnahmen vom Schädel eines Patienten (sog. Ceph-Aufnahmen) enthaltend einen Querträger (9) dessen eines Ende an ein zahnärzliches Röntgendiagnostikgerät (Fig. 1) zur Erstellung von Schichtaufnahmen vom Kiefer eines Patienten adaptierbar ist und an dessen anderem Ende ein Schädelhalter (10) angeordnet ist, der ein um eine lotrechte Achse drehbares Teil (11) enthält, an dem einerseits zwei diametral einander gegenüberliegende Ohrolivenhalter (12) und andererseits ein zumindest in Höhe und Tiefe verstellbares Anlageteil (13) am Nasion des Patienten verstellbar angeordnet sind, und der ferner ein in Richtung auf die Strahlenquelle (4) des Röntgendiagnostikgerätes (Fig. 1) verstellbares Aufnahmeteil (17) für eine Filmkassette (18) enthält dadurch gekennzeichnet, daß der Schädelhalter (10) mittels eines Tragteils (14) am Querträger (9) um eine horizontale Achslagerung (25) derart schwenkbar gehalten ist, daß der Patientenkopf in bezug auf die Sagittalebene in verschiedenen Neigungsstellungen (14') einstellbar ist und der Patientenkopf über ein im Schädelhalter (10) um eine lotrechte Achse drehbar gelagertes Teil (11) so positionierbar ist daß die Kiefergelenke in zwei Ebenen jeweils senkrecht zur Kondylenachse darstellbar sind.

2. Vorrichtung nach Anspruch 1 dadurch gekennzeichnet, daß das Tragteil (14) höhenverstellbar ausgebildet ist.

3. Vorrichtung nach Anspruch 2 dadurch gekennzeichnet, daß das Tragteil (14) aus Teleskoprohren (21, 22) besteht, welche mittels eines Feststellgliedes (24) in verschiedenen Sellungen feststellbar sind.

4. Vorrichtung nach Anspruch 3 dadurch gekennzeichnet, daß die Teleskoprohre (21, 22) von einem Faltenbalg (23) abgedeckt sind.

5. Vorrichtung nach Anspruch 1 dadurch gekennzeichnet, daß das Tragteil (14) mittels eines quer zur Längsrichtung verlaufenden Zapfens (30) an einem am Querträger (9) befestigten Gehäuse (15) schwenkbar gehalten ist.

6. Vorrichtung nach Anspruch 5 dadurch gekennzeichnet, daß der Zapfen als das Tragteil (14) durchdringende Buchse (30) ausgebildet ist, welche um eine mit dem Gehäuse (15) fest verbundenen Lagerzapfen (31) drehbar ist.

7. Vorrichtung nach Anspruch 6 dadurch gekennzeichnet, daß an der Buchse (30) ein einen zur Schwenkachse (25) einen Hebelarm bildendes Teil (34) befestigt ist, welches mittels eines Federelements (33) mit dem Gehäuse (15) im Sinne einer Stabilisierung des Schädelhalters (10) in seiner Grundstellung verbunden ist.

8. Vorrichtung nach einem der Ansprüche 3 bis 7 dadurch gekennzeichnet, daß zur Arretierung des Schädelhalters (10) in den verschiedenen Neigungspositionen eine von Hand betätigbare Spanneinrichtung (26 bis 29) vorgesehen ist.

9. Vorrichtung nach Anspruch 8 dadurch gekennzeichnet, daß die Spanneinrichtung eine das eine Teleskoprohr (22) und eine Gehäusewandung (27) durchgreifende Gewindestange (28) und einen darauf aufgeschraubten Spannhebel (29) enthält und daß die Gewindestange (28) in einer den Neigungswinkel des Schädelhalters (10) begrenzenden Nut (26) geführt ist.

10. Vorrichtung nach einem der Ansprüche 5 bis 9 **dadurch gekennzeichnet**, daß im Gehäuse (15) Führungselemente (36) für zwei die Filmkassettenhalterung (17) tragende Tragstangen (16) vorgesehen sind.

11. Vorrichtung nach Anspruch 10 **dadurch gekennzeichnet**, daß die Tragstangen (16) als Rohre ausgebildet sind und in ihrem Inneren Übertragungselemente (37) drehbar gelagert sind, deren dem Gehäuse (15) abgewandte Enden mit Tasthebeln (39, 40) verbunden sind zur Abtastung der Größe der auf den Filmkassettenhalter (17) aufsetzbaren Filmkassette (18) und deren andere Enden mit Winkelgebern (38) verbunden sind, welche in Abhängigkeit von der Drehstellung der Tasthebel (39, 40) ein vorzugsweise elektrisches Signal als Größe für das gewählte Filmformat liefern.

FIG 1

FIG 2

FIG 3

FIG 4

FIG 5

# EUROPÄISCHER RECHERCHENBERICHT

EP 87 11 3409

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | DE-A-2 335 918 (K.K. MORITA SEISAKUSHO) <br> * Figuren 1,2; Seite 4, Zeile 15 - Seite 7, Zeile 22 * | 1 | A 61 B 6/00 |
| D,A | | 2,5 | |
| | --- | | |
| Y | US-A-4 088 893 (SCHROEDER) <br> * Figur 1; Spalte 2, Zeile 45 - Spalte 3, Zeile 22 * | 1 | |
| A | | 10 | |
| | --- | | |
| A | DE-A-3 221 963 (HANEL) <br> * Figuren 1-3; Seite 7, Zeile 5 - Seite 14, Zeile 9 * | 1,2,5,8 | |
| | --- | | |
| A | US-A-3 790 803 (PHILIPS) <br> * Figuren I,II; Spalte 3, Zeile 15 - Spalte 4, Zeile 4 * | 3 | |
| | --- | | |
| A | US-A-3 643 095 (PSHUSTER) <br> * Zusammenfassung; Figuren 1,3; Spalte 6, Zeilen 6-54 * | 11 | |
| | ----- | | |

**RECHERCHIERTE SACHGEBIETE (Int. Cl.4)**

A 61 B

Der vorliegende Recherchenbericht wurde für alle Patentansprüche erstellt

| Recherchenort | Abschlußdatum der Recherche | Prüfer |
|---|---|---|
| DEN HAAG | 22-12-1987 | CHEN A.H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
....................................................
& : Mitglied der gleichen Patentfamilie, übereinstimmendes Dokument